# EUROPEAN PATENT SPECIFICATION

(11) **EP 1 220 687 B1**
(45) Date of publication and mention of the grant of the patent: **21.02.2007**
(21) Application number: 00964595.3
(22) Date of filing: 27.09.2000
(51) Int. Cl.: A61K 39/395, A61P 35/02, A61P 35/04

(54) **ADMINISTRATION OF GAMMA GLOBULINS TO TREAT LYMPHOMA**
VERABREICHUNG VON GAMMAGLOBULINEN ZUR BEHANDLUNG VON LYMPHOMEN
ADMINISTRATION DE GAMMA GLOBULINES POUR LE TRAITEMENT DU LYMPHOME

(30) Priority: 27.09.1999 US 405050
(43) Date of publication of application: 10.07.2002
(73) Proprietor: ARP BIOMED LTD., Tel Aviv 64332 (IL)
(72) Inventor: SHOENFELD, Yehuda, 52622 Ramat-Gan (IL); FISHMAN, Pnina, 46365 Herzelya (IL)
(74) Representative: Vossius & Partner
(86) International application number: PCT/IL2000/000600
(87) International publication number: WO 2001/022996

(56) References cited:
- US-A- 5 562 902
- BESA E C: "RAPID TRANSIENT REVERSAL OF ANEMIA AND LONG-TERM EFFECTS OF MAINTENANCE INTRAVENOUS IMMUNOGLOBULIN FOR AUTOIMMUNE HEMOLYTIC ANEMIA IN PATIENTS WITH LYMPHOPROLIFERATIVE DISORDERS" AMERICAN JOURNAL OF MEDICINE, vol. 84, no. 4, 1988, pages 691-698, XP000978941 ISSN: 0002-9343
- LUNDIN J ET AL: "CAMPATH-1H monoclonal antibody in therapy for previously treated low-grade non-Hodgkin's lymphomas: A phase II multicenter study." JOURNAL OF CLINICAL ONCOLOGY, vol. 16, no. 10, October 1998 (1998-10), pages 3257-3263, XP000978970 ISSN: 0732-183X

## Description

### TECHNICAL FIELD OF THE INVENTION

The present invention relates to lymphoma therapy and in particular to the administration of gamma globulins. In accordance with this invention, the treatment of lymphoma is accomplished by administering a preparation containing intact gamma globulins or fragments thereof. The gamma globulin preparation can be administered parenterally, preferably via intravenous, or subcutaneous routes, either as a sole drug or in combination wit other treatment regimens which are commonly used for lymphoma treatment.

### BACKGROUND OF THE INVENTION

The formation of metastases of malignant tumors, initiated from a primary tumor at more or less remote locations of the body, is one of the most serious effects of cancer and one for which a satisfactory treatment protocol is currently unavailable. Cancer tumor metastasis is responsible for most therapeutic failures when the disease is treated, as patients succumb to the multiple tumor growth.

The extent to which metastasis occurs varies with the individual type of tumor. Melanoma, lymphoma, breast cancer, lung cancer, colon cancer and prostate cancer are among the types of cancer that are particularly prone to metastasize. When metastasis takes place, the secondary tumors can form at a variety of sites in the body, brain and bone being the more common sites.

The currently available methods of cancer therapy such as surgical therapy, radiotherapy, chemotherapy and other immunobiological methods have either been unsuccessful in preventing metastasis or these methods give rise to serious and undesirable side effects.

In many clinically diagnosed solid tumors (in which the tumor is a localized growth) surgical removal is considered the prime means of treatment. However, many times after surgery and after some delay period, the original tumor is observed to have metastasized so that secondary sites of cancer invasion have spread throughout the body and the patient subsequently dies of the secondary cancer growth. Reports indicate that in individuals with resectable tumors, primary tumor growth or local recurrence is not often the cause of death. Instead, at present, nearly 40% of cancer victims with operable tumors ultimately succumb to metastatic disease following surgery.

Metastasis is a constant occurrence in some tumors. However, many times metastasis is triggered by the surgical operation itself. During the course of surgery malignant cells may become dislodged from the tumor mass and enter the circulatory system thus increasing the chance of metastasis.

Although chemotherapy is widely used in the treatment of cancer, it is a systemic treatment based usually on the prevention of cell proliferation. Accordingly, chemotherapy is a non-specific treatment modality affecting all proliferating cells, including normal cells, leading to undesirable and often serious side effects such as immunosuppression, pancytopenia growth inhibition of bone marrow cells with anemia, thrombocytopenia and leukopenia, diarrhea, nausea and alopecia (hair loss).

Generally, the existing systemic treatments have, quite often, proven to have little effect on micrometastases already residing in remote organs (lung, liver, bone marrow or brain), and they are not very effective in preventing the dissemination of the tumor to other tissues.

Therefore, the need exists for treatments for inhibiting tumor metastasis. In particular, treatments which inhibit metastasis without causing serious side effects are much desired.

Besa, et al., Am J. Med. 1988 Apr. 84(4): 691-8 describes rapid transient reversal of anemia and long-term effect of maintenance intravenous immunoglobulin for autoimmune hemolytic anemia in patients with lymphoproliferative disorders.

US 5,562,902 disclosed the use of IVIG or F(ab')₂ fragments thereof for the preparation of a pharmaceutical composition for inhibiting metastasis of a tumor which is to be administered intravenously.

### SUMMARY OF THE INVENTION

The present invention generally solves the problems referred to above by providing the use of Intravenous Immunoglobulins or fragments thereof, wherein said fragments are F(abs), F(ab'), F(ab')2, Fc or single chains of immunoglobulins for the preparation of a pharmaceutical composition for the treatment of lymphoma in a mammal.

The pharmaceutical composition of this invention do not give rise to serious side effects and will be effective for the treatment of lymphoma in a mammal. In particular, the IVIG preparation to be administered according to this invention may contain intact immunoglobulin molecules or fragments of immunoglobulins. The preparation is administered parenterally, preferably via intravenous, or subcutaneous routes, either as a sole agent or in combination with other treatment regimens which are commonly used for cancer treatment.

### BRIEF DESCRIPTION OF THE DRAWINGS

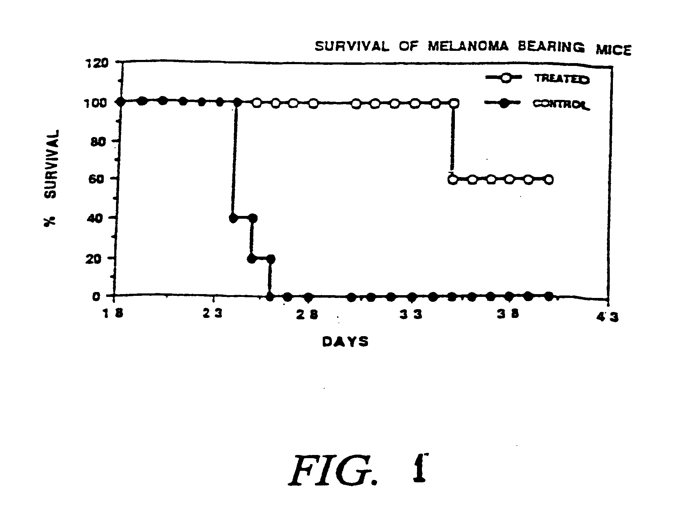

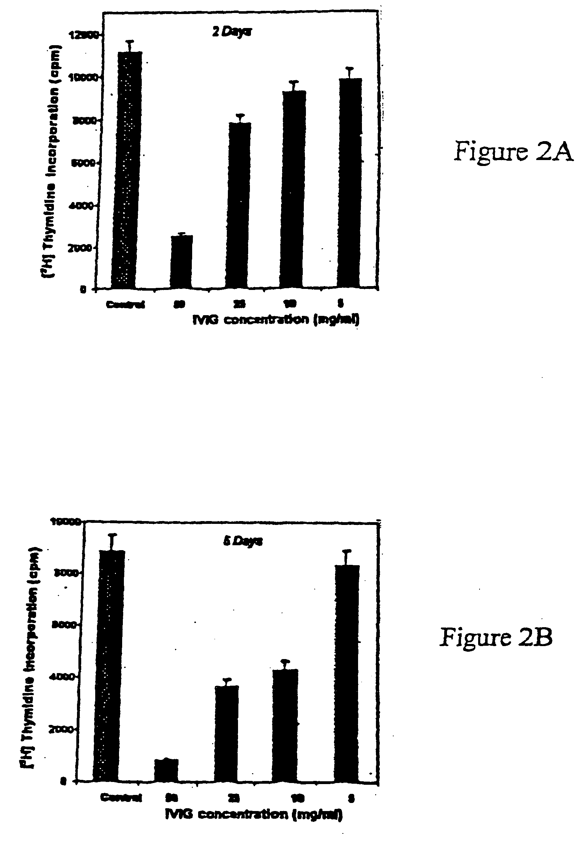

Figure 1 is a graph demonstrating the effect of IVIG administration on the survival of melanoma bearing mice. The mice treated with IVIG had a significantly longer (Reference Example) mean-survival time.Figure 2A is a graph showing the effect of various concentrations of IVIG on the proliferation of Nb2-11c lymphoma cells after 2 days. Figure 2B is a graph showing the effect of various concentrations of IVIG on the proliferation of Nb2-11c lymphoma cells after 5 days. IVIG treatment resulted in dose-dependent inhibition of proliferation of cells at both 2 and 5 days of treatment.

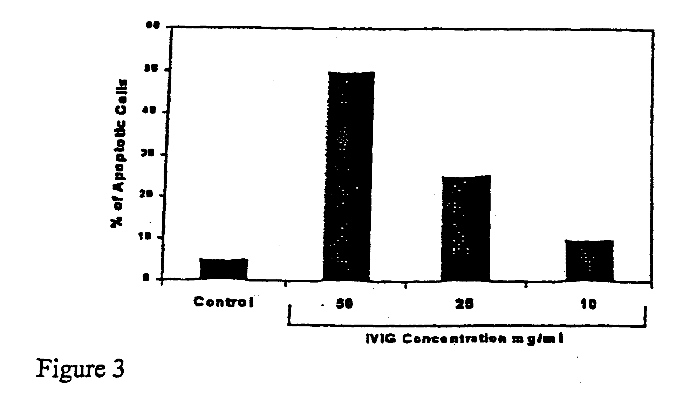

Figure 3 is a graph showing induction of apoptosis in Nb2-11c lymphoma cells with various concentrations of IVIG. IVIG treatment induced apoptosis in a dose-dependent manner.

### DETAILED DESCRIPTION OF THE INVENTION

### Definitions

As used herein, "gamma globulin" is the serum globulin fraction that is mainly composed of IgG molecules.

As used herein, "IVIG" or "intravenous immunoglobulins" refers to gamma globulin preparations suitable for intravenous use, such as those IVIG preparations commercially available from several sources.

As used herein, "fragments" of IVIG or gamma globulin are portions of intact immunoglobulins such as Fc, F(ab), F(ab'), F(ab')₂ and single chain immunoglobulins.

"Metastasis", as used herein, is defined as the transfer of malignant tumor cells, or neoplasm, via the circulatory or lymphatic systems or via natural body cavities, usually from the primary focus of neoplasia to a distant site in the body, and subsequent development of secondary tumors or colonies in the new location.

As used herein, "metastases" means the secondary tumors or colonies formed as a result of metastasis.

As used herein, "inhibition of metastasis" is defined as preventing or reducing the development of metastases. "Intracavitary administration", as used herein, refers to administering a substance directly into a body cavity of a mammal. Such body cavities include the peritoneal cavity, the pleural cavity and cavities within the central nervous system.

Gammaglobulins suitable for intravenous administration are commonly referred to as Intravenous Immunoglobulins) IVIG) and are commercially available from several sources, for example from Miles Inc. (West Haven, Connecticut), N. V. Baxter S.A. (Lessines, Belgium), Sandoz Pharma Ltd. (Basle, Switzerland), Instituto Sierovaccinogeno Italiano (Isiven Italy), Sclavo S.p.A., Siena, Italy and Jackson Immunoresearch Laboratories, Inc. (West Grove, Pennsylvania). The commercially available IVIG preparations contain mainly IgG molecules. IVIG has been used in replacement therapy in primary immunodeficiency syndromes and in secondary immunodeficiencies as well as for the prevention and treatment of infectious diseases. Furthermore, IVIG has also been used for immune modulation of patients with autoimmune and immune-complex diseases. See, Martha M. Eibl, "Intravenous Immunoglobulin: A Review", Immunodeficiency Reviews, 1 (Suppl.), pp. 1-42 (1989).

According to a National Institutes of Health (NIH) Consensus Conference report, the incidence of adverse side effects associated with IVIG use in humans, used at dosage regimens comparable to the ones contemplated by the present invention, is usually less than 5% with most of those reactions being "mild and self-limited." The report adds that "]s[evere reactions occur very infrequently and usually do not contraindicate further IVIG therapy." The NIH report also notes that "[n[either HIV nor hepatitis B infection has been transmitted to recipients of products currently licensed in the United States." NIH Consensus Conference, "Intravenous Immunoglobulin: Prevention and Treatment of Disease", JAMA, 264, pp. 3189-3193 (1990).

The present invention stems from our discovery that IVIG as a whole molecule or the F(ab')₂ fragment by itself, is extremely useful for the treatment of cancerous diseases in murine and rat models.

The gamma globulin preparations that may be used according to the present invention include commercially available preparations of intact IVIG and preparations of the F(ab')₂ fragments of IVIG. Recombinantly produced gamma' globulins and their fragments may also be used according to this invention. The use of recombinant single chain antibodies is also envisioned.

The dosage of IVIG and the administration will vary with the severity and nature of the particular condition being treated, the duration of treatment, the adjunct therapy used, the age and physical condition of the subject of treatment and like factors within the specific knowledge and expertise of the treating physician. However, single dosages for intravenous and intracavitary administration can typically range from 400 mg to 2 g per kilogram body weight, preferably 2 g/kg (unless otherwise indicated, the unit designated "mg/kg" or "g/kg", as used herein, refers to milligrams or grams per kilogram of body weight). The preferred dosage regimen is 400 mg/kg/day for 5 consecutive days per month or 2 g/kg/day once a month. The IVIG according to the present invention was found to be effective in inhibiting metastasis in animal models when administered by intravenous or intraperitoneal injection and in the dose range of 500-1000 mg/kg/week.

In another embodiment of this invention, the IVIG preparation is to be administered via the subcutaneous route. The typical dosage for subcutaneous administration can range from 4 mg to 20 mg per kg body weight. The IVIG according to the present invention was found to be effective in inhibiting metastasis in mice when administered subcutaneously in the dose 200 µg/mouse. According to the present invention IVIG may be administered as a pharmaceutical composition containing a pharmaceutically acceptable carrier. The carrier must be physiologically tolerable and must be compatible with the active ingredient. Suitable carriers include, sterile water, saline, dextrose, glycerol and the like. In addition, the compositions may contain minor amounts of stabilizing or pH buffering agents and the like. The compositions are conventionally administered through parenteral routes, with intravenous, intracavitary or subcutaneous injection being preferred.

The intravenous immunoglobulins administered according to the present invention act as antimetastatic agents resulting in the reduction of tumor colony number as well as tumor colony size. They can also act prophylactically i.e., to prevent metastasis of tumors. The intravenous immunoglobulins according to this invention may also be used to reduce the size of the primary tumor.

The treatment described in the present invention may also be used either preceding or subsequent to a surgical procedure to remove the primary tumor. Frequently, metastasis of tumor cells will occur as a result of the physical manipulation of the tumor during surgery. However, the use of the treatment described in the present invention in conjunction with (i.e., before, during and/or after) surgery will reduce the risk of metastasis and consequently this combination of methods would be a more attractive treatment option for the complete elimination of cancerous tumors.

Similarly, other treatment modalities such as chemotherapy, radiation therapy and immunotherapy may also be used in conjunction with (i.e., before, after or at the same time as) the pharmaceutical compositions of the present invention.

Although not wishing to be bound by any particular theory, we propose that intravenous immunoglobulins inhibit metastasis according to one or more of the following mechanisms.

It is known that tumor metastasis occurs following a detachment of single cancerous cells from the tumor, their migration to adjacent or distal tissues, and their seeding and homing in the new organ. The migration process takes place through adhesion molecules which enable the tumor cells to adhere to the blood vessel wall, to penetrate the blood stream and then to emerge and seed in another tissue. We assume that when whole IVIG or the F(ab')₂ fragments of IVIG are applied, they may interfere with the binding of adhesion molecules responsible for the transmission of the tumor cell to and from the blood vessel, and thus prevent the dissemination of the tumor cells to other tissues in the body.

Another possible mechanism is the presence of antibodies or anti-idiotypes in the IVIG mixture which can bind to the tumor cells and induce their lysis in the presence of complement or enhance entrapment of the tumor cells by Fc receptors on the reticuloendothelial system (RES).

An additional possibility is that intravenous immunoglobulins reduce or prevent metastasis by increasing the efficiency of the immune system through inducing the secretion of cytokines such as tumor necrosis factor and interferon gamma.

In the treatment of lymphoma, recent experiments indicate that IVIG acts to induce apoptosis (programmed cell death) in a T-cell lymphoma cell line in vitro.

We are currently conducting additional experiments to elucidate the exact mechanism by which IVIG preparations exert their observed effects on tumor metastasis.

The effect of IVIG on the dissemination of tumors according to the present invention is demonstrated by the following examples carried out in murine and rat models of melanoma, sarcoma and lymphoma. Additionally, we also present clinical data of a representative human melanoma patient, treated with IVIG. These examples are set forth so that this invention may be better understood and are not to be construed as limiting its scope in any manner.

### EXAMPLES

### MATERIALS AND METHODS

### Tumor Cells:

Tumor cell lines from murine origin were used. The murine cell lines included: MCA-105, a methylcholanthrene-induced sarcoma of C57BL/6J origin and B16-F10 melanoma cells (both lines were purchased from American Type Tissue Culture Collection, Rockville, MD). The cells were routinely maintained in RPMI medium containing 10% fetal calf serum. Twice a week the cells were transferred to a freshly prepared medium.

### Experimental Animal Models:

2-3 months old C57BL/6J mice were used during the study. To examine the efficacy of gamma globulin in vivo, 2 types of solid tumors were induced in C57BL/6J mice, e.g. sarcoma (MCA-105) and melanoma (B16nF10). The tumor cells were induced either by intravenous (IV) injection which led to their seeding and lodging in the lung or by intraperitoneal (IP) induction where the cancerous cells developed local lesions in the peritoneum. Some of the mice were sacrificed following 3-5 weeks and examined for metastatic foci in the lungs or spread of tumors in the peritoneum. In another group of mice, survival time was observed. Mice that were injected with tumor cells by IV mode were treated by IV infusion of gamma globulin, whereas in animals in whom the tumor was induced directly in the peritoneum, the gamma globulin preparation was administered through IP injection.

### Gamma Globulin Preparations:

Human gamma globulin suitable for intravenous use (IVIG) was obtained from Miles Inc. (Biological Products Division, West Haven, Connecticut). A 5% solution (5gr in 100ml diluent; Catalogue No. 0640-12) was used for all experiments. Unless otherwise indicated, the volume of IVIG inoculated was 500µl per animal on each treatment which amounted to 25 mg of gamma globulin per animal. Other preparations used were a whole molecule human gamma globulin or an F(ab')₂ fragment both purchased from Jackson Immunoresearch Laboratories, Inc., West Grove, PA, (Code Numbers 009-000-003 and 009-000-006 respectively). These latter preparations are different from the one obtained from Miles Inc. in that they are prepared from a donor pool of 30 whereas the Miles preparation is from a donor pool of 3000 or more individuals.

### Reference Example 1: Effect of Gamma Globulin on the Development of Metastatic Melanoma in C57BL/6J Mice

An experimental model for metastatic melanoma was established using the B16-F10 mouse melanoma cell line. The induction of the melanoma was carried out by IV injection of the tumor melanoma cells which are subsequently seeded in the mice lung and form black metastatic foci. Approximately 24 days following tumor inoculation, the mice die.

In the present experiment the mice were injected either with 2x10⁵ tumor cells or with 5x10⁵ cells and were treated intravenously with IVIG (Miles). The mice were sacrificed on day 18 and the efficacy of the treatment was determined by counting the number of the black metastatic foci in the lungs of the animals.

### A. Inoculation of mice with 2x10⁵ B16-F10 melanoma cells.

20 mice were IV injected with 2x10⁵ melanoma cells and were divided into 4 groups:
(a) Control group, mice inoculated with tumor cells only;
(b) The mice treated with one IV injection of IVIG on day 0 (the day of tumor administration);
(c) The mice treated 2 times, on day 0 and on day 4; and
(d) The mice treated 3 times on days 0, 4 and 9.

The mice were sacrificed on day 18 and the number of the metastatic foci in the lungs was evaluated. Table I summarizes the results. One treatment reduced the number of metastatic foci by 80%, while no foci could be detected following two or three treatments.

**TABLE I**

| Reduction in number of metastatic foci in the lung of mice injected with 2x10⁵ B16-F10 melanoma cells and then treated with IVIG. | |
|---|---|
| GROUP | NO. OF FOCI |
| Control | 20+4 |
| 1 Treatment | 4+2 |
| 2 Treatments | 0 |
| 3 Treatments | 0 |

Black metastatic foci are seen in the control group, less foci in the group that was treated with 1 injection of IVIG and none is seen in the lungs that were derived from mice treated by two or three injections of IVIG.

### B. Inoculation of mice with 5x10⁵ B16-F10 melanoma cells

In order to explore whether IVIG is capable of preventing metastasis when a larger mass of melanoma was involved, the following experiment was conducted. Mice were injected with an increased number of tumor cells (5x10⁵) and divided into 2 groups:
(a) Control group, inoculated with tumor cells only; and
(b) mice treated with 2 IV injections of IVIG on day 0 and on day 8 following tumor inoculation.

On day 18, the mice were sacrificed. Evaluation of the lung metastatic foci revealed a marked decrease in their number in the IVIG treated group. The results are summarized in Table II.

**TABLE II**

| Reduction in number of metastatic foci in the lungs of mice injected with 5x10⁵ B-16 F10 melanoma cells and then treated with IVIG. | |
|---|---|
| GROUP | NO. OF FOCI |
| Control | 165±13.4 |
| Two Treatments | 16.3±3.9 |

There is about a 90% reduction in the number of foci in the treated mice when compared to the control group. These results show that IVIG is capable of inhibiting metastatic spread of melanoma even when a larger tumor mass is involved.

### C. Effect of Gamma Globulin on the Survival of Melanoma Bearing Mice:

4x10⁵ B16-F10 cells were injected IV to C57BL/6J mice. The mice were treated from day 0 and every 7th day thereafter with 500µl Miles IVIG. 24 days following the inoculation of the tumor, the mice from the control group began to die. The results are summarized in Figure 1. While on day twenty six 100% of the control mice were dead, 100% of the IVIG treated mice were alive. On day 40, 60% of the treated mice were still alive.

### Reference Example 2: Effect of Gamma Globulin on the Development of MCAn105 sarcoma in C57BL/6J mice:

MCA-105 cells are derived from mice that developed tumors following methylcholanthrene administration. Two types of experiments were carried out using these cells:
1. Tumor induction by IV infusion:2.5x10⁵ MCA-105 cells were injected IV to the tail vein of C57BL/6J mice. One group of mice was treated by IV infusion of IVIG on days 0, 7, and 14 and the mice belonging to the control group were injected on the same days with phosphate buffered saline (PBS).
   40 days later the mice were sacrificed and their lungs were evaluated for tumor lesions. The lungs of the control group of mice were much larger than those of the IVIG treated animals and were covered with metastatic foci which appeared as white "blebs".
2. Tumor induction by IP infusion:
   2.5x10⁵ MCA-105 cells were injected IP to C57BL/6J mice. The mice were treated with IVIG from day 0 and every 7th day thereafter till they were sacrificed on day 48. Large tumor foci were observed in the peritoneum in the control group of mice whereas in the treated animals only few small foci were seen.

### Reference Example 3: Comparison of the Effect of Intact Gamma globulin and F(ab')₂ fragment on the development of B-16 melanoma in C57BL/6J mice:

Mice were IV inoculated with 2.5x10⁵ B16-F10 melanoma cells. The mice were divided into 3 groups:
a) A control group;
b) mice treated with whole molecule IVIG (Jackson Immunoresearch Laboratories Inc.); 5 mg in a volume of 330 µl was injected IV on days 0, 3, 7 and 12; and
c) a group of mice that was treated with 5 mg of F)ab'(₂ fragment of IVIG )Jackson) in a volume of 500 µl on the same days as in (b(.

The mice were sacrificed on day 17 and black metastatic foci were counted in the lung. In the control group 160±18 metastatic foci were counted in comparison to 68±12 in the group treated with the preparation of intact IVIG, and 13±4 foci in the mice treated with the preparation of F(ab')₂ fragments of IVIG. This result indicates that both intact IVIG and their F(ab')₂ fragments are effective in inhibiting metastases. The observed difference in the effectiveness between the intact IVIG and the F)ab (₂ preparation is probably due to the difference in the specific activities of the two solutions used. The difference between the results of Example 1 where the mean number of metastatic foci was 4 when intact IVIG were administered (Table I) and the present example where the mean number of foci was 68 is probably due to the fact that in Example 1, 25 mg of whole IVIG was injected on day 0, whereas in this example only 5 mg was injected on day 0.

### Reference Example 4: Effect of Gamma Globulin in the Inhibition of Metastasis of Melanoma following Surgical Removal of Primary Tumor:

C57BL6J mice were injected in the foot pad with 2.5x10⁵ B16F10 melanoma cells. After 21 days the leg in which tumor developed was amputated. On the same day the mice were divided into two groups, one group was treated by intravenous injection of 25 mg IVIG ) Sandoz Pharma. Ltd., Basle, Switzerland; Lot - 4.372.256.0) and the other group with phosphate buffered saline (PBS). Ten days later, the mice were examined for signs of tumor development. 60% of the control group developed tumor with a mean size of 3 ± 0.8 cm. Those mice died during the first month following amputation. In the IVIG treated group, only 14% of the mice developed tumor (mean size 2.7 ± 1.2 cm). These mice also died during the first month following amputation. The remaining 86% of the IVIG treated mice did not develop tumor and were still alive 45 days following surgery.

### Reference Example 5: Effect of Low Dose, Subcutaneous Administration of Gamma Globulin in the Inhibition of Melanoma Metastasis:

C57BL6J mice were injected intravenously with 2.5 x 10⁵ B16 melanoma cells per mouse. Immediately thereafter, the mice were administered IVIG preparations via the subcutaneous route in the chest area. Four groups of mice (20 mice/group) received 200 µg/mouse of one of the following commercially available IVIG preparations obtained from Baxter )Gammagard S/D 2.5%; Lot-93H23AB12C(, Isiven )Isiven V.I. 2.5%; Lot-IS238C6193V), Miles )Gamimmune N 5%; Lot-640N023) and Sandoz )Lot-4.372.256.0). A. fifth group of mice receiving intravenous PBS administration acted as the control group. The mice were sacrificed 18 days later and their lungs examined for the presence of metastatic foci. The following table depicts the results.

**TABLE III**

| GROUP | NO. OF FOCI | % INHIBITION |
|---|---|---|
| Control | 50 | - |
| Baxter | 18.6 | 62.8 |
| Miles | 28.5 | 43.0 |
| Isiven | 18.1 | 63.8 |
| Sandoz | 27.9 | 44.2 |

As shown above, low dose, subcutaneous IVIG administration inhibited melanoma metastasis by an average of 53.45% when compared to mice treated with PBS.

### Reference Example 6: Protocol for Use of Gamma Globulin to Inhibit

Metastasis in Human Cancer Patients: IVIG preparations are parenterally administered to cancer patients, generally using one of the following dosage regimens for intravenous and intracavitary administration: (1) 400 mg/kg per day for 5 consecutive days per month or (2) 2 g/kg once a month. For subcutaneous use, the IVIG preparation may be administered in the dose of 4 mg/kg per day for 5 consecutive days per month or 20 mg/kg once a month. However, these suggested regimens may be varied according to the patient's age and physical condition, and the severity of disease. The exact protocol will be determined by the treating physician, taking into consideration various factors and circumstances of each patient. Following administration of the gamma globulin preparation, the patient's progress will be monitored according to standard medical procedure. Additionally, the patient will be examined for tumor metastasis or regression.

### Reference Example 7: Effect of Intravenous Administration of Gamma Globulin to a Representative Melanoma Patient:

A forty-two year old male underwent surgery in September 1989 for the wide excision of a malignant melanoma lesion (depth 1.3 mm) on his left thigh. In May 1991, he underwent a hyperthermic perfusion of the leg with cisplatinum because of a local recurrence of the melanoma in a left femoral lymph node. At that time there was no evidence of metastatic disease in the patient. In February 1993, computerized tomography (CT) scans of the chest and abdomen revealed mass lesions in the spleen (one lesion), the liver (five lesions, the largest being 3x3 cm) and the lungs (four lesions, the largest being 1.5x1.5 cm). Despite the lesions, the patient was.asymptomatic. Soon thereafter, a treatment with IVIG was begun. The patient was intravenously administered IVIG (Miles) at a dose of 400 mg/kg/day for 5 consecutive days per month. After five cycles of treatment, all of the spleen and liver metastases disappeared and there was also a slight reduction in the lung lesions. Afterwards, the patient's condition deteriorated with the appearance of new bone and subcutaneous lesions. He continued to receive IVIG with minor reemergence of liver metastases. The patient expired after receiving 12 cycles of IVIG.

As will be appreciated by one of skill in the art, large liver and spleen metastases of melanoma do not regress spontaneously. Furthermore, it is well known that after the detection of such large metastases, the survival time of patients is usually no longer than a few months.

### Example 8: Effect of Gamma Globulin on T-cell lymphoma cells in vitro

### MATERIALS AND METHODS

### Cell Proliferation Assays

The rat Nb2-11C T-cell lymphoma cell line was used (Pines et al., "Inhibition of the proliferation of Nb2 cells by femtomolar concentrations of cholera toxin and partial reversal of the effect by 12-O-tetradecanoyl-phorbol-13-acetate," J. Cell. Biochem., 37, pp. 119-129 (1988). Cells (1.2 x 10⁴/well) were incubated with RPMI medium containing 5% fetal bovine serum (FBS) in 96 microtiter plates for 48h. IVIG (Sclavo S.p.A., Siena, Italy) was added to the cell cultures at concentrations of 50, 25, 10 and 5 mg/ml for 48 and 120 hours. Cultures containing cells suspended in RPMI medium and 5% FBS served as controls.

During the last 18 hours of incubation, each well was pulsed with 1µCi ]³H[-thymidine. The cells were harvested and the ]³H[ -thymidine uptake was determined in a LKB liquid scintillation counter (LKB, Piscataway, NJ, USA (.

### Cell Cycle Analysis

Flow cytometric analysis of the cell cycle of Nb2-11C cells was carried out by propidium iodide staining. A sub-G1 peak by FACS analysis represents the apoptotic cells and the percentage of these among the whole cell population can be analyzed. Cells, at a concentration of 1.2x10⁵/ml, were cultured in RPMI medium and IVIG at a concentration of 50, 25, 10 and 5 mg/ml and incubated for 48 h at 37°C. Cells cultured in RPMI and 5% FBS served as control. At the end of the incubation period, the cells were washed three times with PBS and resuspended in staining buffer containing 0.1% bovine serum albumin (BSA), 50 g/ml propidium iodide, 0.1% Triton X-100 and 1mg/ml RNAase )boiled for 10 min). Samples were examined after 30 min of staining on a FACScan flow cytometer (Becton Dickinson & Co., Mountain View, CA, USA).

### Detection of Apoptosis by Acridine Orange Staining

Acridine orange staining was performed according to Hare and Bahler, "Analysis of plasmodium falciparum growth in culture using acridine orange and flow cytometry," J. Histochem. Cytochem., 34, pp. 215-220 (1986). Cytospin preparations were made directly on slides from Nb2-11C cells treated with 25 mg IVIG. Slides were fixed with 100%' ethanol for 10 minutes. Acridine orange at 1.2µg/ml was dissolved in citrate/EDTA buffer (0.13M Na₂HPO₄, 0.35M citric acid and 1 M Na₂EDTA, pH 6.5) and applied on the slides for 30 minutes. Cells were examined and counted under a fluorescence microscope (Olympus BH-2(.

### RESULTS

Proliferation of Nb2-11C cells was inhibited in a dose-dependent manner following incubation with various IVIG concentrations (Figures 2A and 2B). The cell proliferation inhibition was a result of apoptosis rather than a cell cycle arrest, because cell cycle analysis revealed significant levels of apoptotic cells in IVIG treated cultures (Figure 3). The percentage of apoptosis was directly related to the IVIG concentration (i.e., the highest degree of apoptosis was observed at a concentration of 50 mg/ml). These results were confirmed by acridine orange staining, where apoptotic bodies were seen in the IVIG-treated culture. In the untreated culture, only well-preserved nuclei were seen.

It will therefore be appreciated that IVIG, at concentrations similar to those found in the blood of patients treated with IVIG, is capable of inducing apoptosis in a T-cell lymphoma cell line.

While we have described above specific examples of this invention, it will be apparent to those skilled in the field of cancer therapy that our basic methods may be altered according to need. Therefore, it will be appreciated that the scope of our invention is to be defined by the claims appended hereto rather than by the specific embodiments presented hereinbefore by way of example.

## Claims

1. The use of Intravenous Immunoglobulins or fragments thereof, wherein said fragments are F(ab), F(ab'), F(ab')2, Fc or single chains of immunoglobulins for the preparation of a pharmaceutical composition, for the treatment of lymphoma in a mammal.

2. The use according to claim 1, wherein the Intravenous Immunoglobulins or fragments thereof is administered intravenously.

3. The use according to claim 1, wherein the Intravenous Immunoglobulins or fragments thereof is administered subcutaneously.

4. The use according to anyone of claims 2-3, wherein administration of the preparation of Intravenous Immunoglobulins or functional fragments thereof precedes, coincides or follows a second treatment regimen wherein the second treatment regimen is chemotherapy, immunotherapy, radiation therapy or surgery.

5. The use according to claim 4, wherein said surgery involves the removal of the solid tumor.

6. The use according to claim 1, wherein said fragment of Immunoglobulin is F(ab')2 fragment.

## Patentansprüche

1. Verwendung von intravenösen Immunglobulinen oder Fragmenten davon für die Herstellung eines Arzneimittels zur Behandlung von Lymphoma bei einem Säuger, wobei die Fragmente F(ab), F(ab'), F(ab')₂, Fc oder Einzelketten von Immunglobulinen sind.

2. Verwendung gemäß Anspruch 1, wobei die intravenösen Immunglobuline oder Fragmente davon intravenös verabreicht werden.

3. Verwendung gemäß Anspruch 1, wobei die intravenösen Immunglobuline oder Fragmente davon subkutan verabreicht werden.

4. Verwendung gemäß einem der Ansprüche 2 bis 3, wobei die Verabreichung des Präparats von intravenösen Immunglobulinen oder funktionellen Fragmenten davon einem zweiten Behandlungsverfahren vorangeht, gleichzeitig erfolgt oder sich an dieses anschließt, wobei das zweite Behandlungsverfahren Chemotherapie, Immuntherapie, Radiotherapie oder chirurgische Therapie ist.

5. Verwendung gemäß Anspruch 4, wobei die chirurgische Therapie die Entfernung des soliden Tumors beinhaltet.

6. Verwendung gemäß Anspruch 1, wobei das Fragment des Immunglobulins F(ab')₂ ist.

## Revendications

1. Utilisation d'immunoglobulines intraveineuses ou de fragments de celles-ci, dans laquelle lesdits fragments sont des F(ab), F(ab'), F(ab')2, Fc ou des chaînes uniques d'immunoglobulines pour la préparation d'une composition pharmaceutique, pour le traitement d'un lymphome chez un mammifère.

2. Utilisation selon la revendication 1, dans laquelle les immunoglobulines intraveineuses ou les fragments de celles-ci sont administrés en intraveineuse.

3. Utilisation selon la revendication 1, dans laquelle les immunoglobulines intraveineuses ou les fragments de celles-ci sont administrés en sous-cutané.

4. Utilisation selon l'une quelconque des revendications 2-3, dans laquelle l'administration de la préparation d'immunoglobulines intraveineuses ou des fragments fonctionnels de celles-ci précède, coïncide ou suit un second régime de traitement, le second régime de traitement étant une chimiothérapie, une immunothérapie, une radiothérapie ou une chirurgie.

5. Utilisation selon la revendication 4, dans laquelle ladite chirurgie implique l'ablation de la tumeur solide.

6. Utilisation selon la revendication 1, dans laquelle ledit fragment d'immunoglobuline est un fragment F(ab')2.
